# EUROPEAN PATENT APPLICATION

(11) **EP 4 122 448 A1**
(43) Date of publication of application: **25.01.2023**
(21) Application number: 21784347.3
(22) Date of filing: 31.03.2021
(51) Int. Cl.: A61K 9/08, A61K 9/00, A61K 47/20, A61K 47/26, A61K 47/12, A61K 47/22, A61K 38/48, A61M 5/31, A61M 5/28

(54) **BOTULINUM TOXIN PRE-FILLED INJECTABLE FORMULATION THAT FACILITATES DISCHARGE RATE CONTROL AND IS STABLE**

(30) Priority: 08.04.2020 KR 20200042962; 08.04.2020 KR 20200042966; 08.04.2020 KR 20200042967
(71) Applicant: Medytox Inc., Chungcheongbuk-do 28126 (KR)
(72) Inventor: RHEE, Chang Hoon, Seoul 04572 (KR); YUN, Ji Hyun, Sejong 30098 (KR); PARK, Young Su, Sejong 30101 (KR)
(74) Representative: Brevalex
(86) International application number: PCT/KR2021/003989
(87) International publication number: WO 2021/206360

(57) **Abstract**

Provided is a botulinum toxin pre-filled injectable formulation that facilitates discharge rate control and is stable.

## Description

### TECHNICAL FIELD

The present disclosure relates to a syringe formulation that is pre-filled with botulinum toxin, which allows easy control of a discharge rate thereof and is stable.

### BACKGROUND ART

Botulinum toxin (BoNT) is one of the most powerful toxins known and acts by blocking the release of acetylcholine in peripheral cholinergic neurons. Botulinum toxin is administered to individuals in very small doses, to reduce overactive muscles and hyperactive exocrine glands. For example, botulinum toxin is being used for cosmetic purposes as well as for the treatment of neuromuscular diseases and hyperactive exocrine gland diseases.

Botulinum toxin is inherently unstable, and is known to be highly unstable and heat-labile, especially at alkaline pH. The medical form of administration that overcomes these shortcomings is the pre-filled syringe method, which has become increasingly popular as a drug delivery device in recent years. However, when a protein is used as an active ingredient, due to the limited stability of the protein, it is a particularly difficult task for formulation scientists to use the pre-filled syringe method. In particular, such difficulties apply to very dilute aqueous solutions of botulinum toxin.

In order to increase the stability of pharmaceutical botulinum toxin compositions, stabilizing proteins such as human serum albumin (HSA) are often added. Also known is the addition of non-proteinaceous stabilizers such as surfactants, polyvinylpyrrolidone (PVP), disaccharides, and polyols.

However, even in view of the aforementioned background art, there is still a demand for botulinum toxin pre-filled syringe formulations that have improved storage stability, allow easy control of a discharge rate thereof, and are stable.

### DESCRIPTION OF EMBODIMENTS

### TECHNICAL PROBLEM

According to an aspect, a syringe formulation that is pre-filled with botulinum toxin is provided, which allows easy control of a discharge rate and may be stable.

### SOLUTION TO PROBLEM

According to an aspect, a syringe formulation pre-filled with botulinum toxin is provided, wherein the syringe formulation may include a syringe including a syringe barrel, a plunger rod, and a plunger stopper, and a botulinum toxin liquid preparation filled in the syringe.

In the syringe formulation, an amount of increase of a force acting on an end of the plunger rod may decrease as a rate of discharge of the botulinum toxin liquid preparation increases.

The term "pre-filled syringe" as used herein refers to a syringe having a partially or completely enclosed space, which may be sealed or sealed and may be used to accommodate, store, and/or transport liquid formulations. The syringe may be a closed or sealed container made of plastic such as an organic polymer, or partially or mainly made of plastic.

The pre-filled syringe may have 2 openings sealed to prevent leakage of contents. The pre-filled syringe has a proximal end sealed by a plunger stopper and the distal end is sealed by a capping device.

The barrel may include a proximal end and a distal end, and a wall that may extend therebetween and is generally cylindrical to form a barrel lumen. The barrel may have a tip protruding to the distal having a fluid passage extending therethrough and in communication with the barrel lumen. The proximal end and the distal end respectively have a proximal end open outlet and a distal end open outlet. The tip may have the same material as the rest of the barrel.

The barrel is removably capped after filling, by a capping device for sealingly closing the distal end of the syringe (e.g., by a "tip cap" that may be removed before use and replaced with a needle, or by a sealer such as a needle shield in the case of a pre-filled syringe having a removable or permanent needle), and is closed at the proximal end by a plunger or by any other unit fluid-tightly-engaged with the inner wall of the barrel to accommodate the fluid. To use a pre-filled syringe, a tip cap, a needle shield, or other type of capping device, may be removed, optionally the needle (if not already present) may be attached, and the plunger tip or piston may be moved forward into the barrel to inject the contents in the barrel, i.e., a botulinum toxin preparation, into a patient.

An inner surface of the barrel may or may not be coated. The inner surface of the barrel may be coated, for example, with a barrier layer (hereinafter referred to as a "lubricant layer") for lubrication. The lubricant layer not only provides a high lubrication that allows the plunger to glide easily through the barrel, but also is compatible with the botulinum toxin formulation and ensure the storage lifespan of the botulinum toxin formulation. The lubricant layer may be a silicone-free lubricant layer or a silicone lubricant layer. The inner surface of the wall may be coated with silicone. That is, the barrier layer may be a silicone layer.

The silicone coating may be prepared by a method selected from a silicone oilbased method (e.g., spray-on siliconization or baked-on siliconization) and a deposition method (e.g., plasma-enhanced chemical vapor deposition (PECVD)). The silicone coating may be formed, for example, by spray-on siliconization or by a baked-on siliconization coating. The silicone coating may be a polydimethylsiloxane coating.

In the spray-on siliconization method, for example, a diving or static nozzle may be used to spray silicone oil (e.g., DOW CORNING 360 with a viscosity of 1,000 cSt) into a syringe, i.e., a barrel, to produce a thin silicone oil layer. The baked-on siliconization method may include applying silicone oil to a barrel as an emulsion, for example, a DOW CORNING 365 siliconization emulsion, and then baking on the plastic surface for a certain time at a certain temperature. The baked-on siliconization method may result in less silicone oil particles invisible to the naked eye and less silicone oil particles visible to the naked eye than the spray-on siliconization method.

A material of the syringe barrel may include a plastic material. The material may be glass, COC, COP, or a mixture thereof. A surface of the COP may be silicone-coated. COC is produced by polymerization of ethane with a cyclic monomer such as norbornene. Also, COC may be produced by hydrogenation after ring-opening metathesis of a cyclic monomer. COC, COP, and a mixture material of COP and COC may exhibit several desirable features, including high transparency, low density, excellent moisture barrier ability, and resistance to aqueous and polar organic media. Detailed examples thereof include Topas^{®} COC and the DaikyoCrystal Zenith^{®}.

The tip of the barrel may be integral with the barrel. For example, the tip may be molded into an integral with a barrel. The barrel may include an integrally formed luer lock tip or a luer slip tip. The tip may extend in an axial direction through the tip and may include an integral passage in communication with a chamber to distribute the contents of the barrel. The tip may have a substantially frustum cone shape that converges from the distal outlet end of the barrel towards the outlet end of the tip.

The barrel may be, for example, having an inner diameter adjusted to accommodate a filling volume of 0.5 mL, 1.0 mL, 1.5 mL, or 2.0 mL. The barrel may have a scale indication indicating a volume of fluid in a syringe. In addition, the barrel may include a flange-style interface. The design of the flange may be, for example, compatible with ISO11040. The flange-style interface may be compatible with an optional handle.

The ratio of the length to the inner diameter of the syringe barrel may be in a range of 10 to 22, 12 to 20, or 14 to 16.

The inner diameter of the syringe barrel may be in a range of 3 mm to 7 mm, 3.5 mm to 6.5 mm, 4 mm to 6 mm, or 4.5 mm to 5.5 mm, and the length of the syringe barrel may be in a range of 30 mm to 130 mm, 50 mm to 110 mm, 60 mm to 100 mm, or 70 mm to 90 mm. The inner diameter of the barrel of the syringe may be in a range of 3.5 mm to 6.5 mm, and the length of the barrel may be in a range of 60 mm to 100 mm. In addition, the inner diameter of the barrel of the syringe may be in a range of 3 mm to 7 mm, and the length of the barrel may be in a range of 30 mm to 130 mm. In addition, the inner diameter of the barrel of the syringe may be in a range of 4 mm to 6 mm, and the length of the barrel may be in a range of 50 mm to 110 mm. In addition, the inner diameter of the barrel of the syringe may be in a range of 4.5 mm to 5.5 mm, and the length of the barrel may be in a range of 70 mm to 90 mm.

The plunger rod and the plunger stopper may be combined to form a plunger rod and a plunger stopper assembly. The assembly may extend into the proximal end of the barrel. The assembly may include a rod having a plunger stopper at a tip thereof, which may be in sliding fluid-tight engagement with a cylindrical wall of the barrel lumen. The assembly may form a proximal sealing portion and a dynamic sealing portion that may enable extrusion of the botulinum toxin liquid preparation. The stopper may be in contact with the botulinum toxin liquid preparation during storage and/or administration.

The stopper may be prepared by using an elastomer material. The stopper may optionally have a coating on at least a portion thereof that is in contact with the botulinum toxin liquid preparation during storage and/or injection.

The elastomer may be isoprene rubber (IS), butadiene rubber (polybutadiene, BR), butyl rubber (copolymer of isobutylene and isoprene, IIR), halogenated butyl rubber (e.g., chlorobutyl rubber, CIIR; and bromobutyl rubber, BIIR), styrene-butadiene rubber (copolymer of styrene and butadiene, SBR), or a mixture thereof. In one embodiment, the stopper material may be butyl rubber, halogenated butyl rubber, or a mixture thereof. In one embodiment, the stopper material may be bromobutyl rubber or chlorobutyl rubber. The elastomer may also be reinforced by an inert mineral. In addition, the elastomer may be cured, for example, by organic peroxides, phenolic resins, and the like.

The stopper may be coated or uncoated. The coating may be normally applied to at least a surface of the sealing portion, which may be facing the barrel lumen and may include a surface portion of the plunger stopper in contact with the botulinum toxin formulation during storage and/or use. The coating may provide satisfactory lubricity while minimizing interaction between the plunger stopper and the botulinum toxin liquid preparation.

A suitable coating of the plunger stopper generally may not undesirably interfere with the botulinum toxin formulation and may be prepared by using a material that may show a low level of extractables/leachables. The coating may include polypropylene, polyethylene, parylene (e.g., parylene N, parylene C, and parylene HT), crosslinked silicone, fluoropolymer, or a mixture thereof. Examples of crosslinked silicone coatings include B2-coating (Daikyo Seiko) or XSi^{™} (Becton Dickinson).

The fluoropolymer coating may be a fluorinated ethylene-propylene copolymer (e.g., a tetrafluoroethylene-hexafluoropropylene copolymer (FEP)), a fluorinated ethylene-ethylene copolymer (e.g., ethylene tetrafluoroethylene copolymer (ETFE), such as Flurotec^{®}), PVA (a copolymer of tetrafluoroethylene (TFE) and perfluoropropyl vinyl ether (PPVE)), a tetrafluoroethylene-perfluoroethylene copolymer, a polyvinylidene fluoride (PVDF), a polyvinyl fluoride (PVF), polytetrafluoroethylene (PTFE) (Teflon), or a mixture thereof. For example, the coating may be prepared by using ETFE, in particular, Flurotec^{®} coating. In one embodiment, the plunger stopper may be uncoated.

The design of the plunger stopper is not particularly limited and may be a nested or bagged stopper. In addition, a thread may be formed at an interface with the rod to enable installation of the rod after sterilization. Alternatively, the interface with the rod may be designed in a snap-on design. A rod, such as a plunger stopper, may be generally designed to withstand sterilization, but may not be otherwise limited in any particular manner. The rod may be prepared by using a plastic material such as ethylene vinyl acetate (EVA) copolymer or polypropylene.

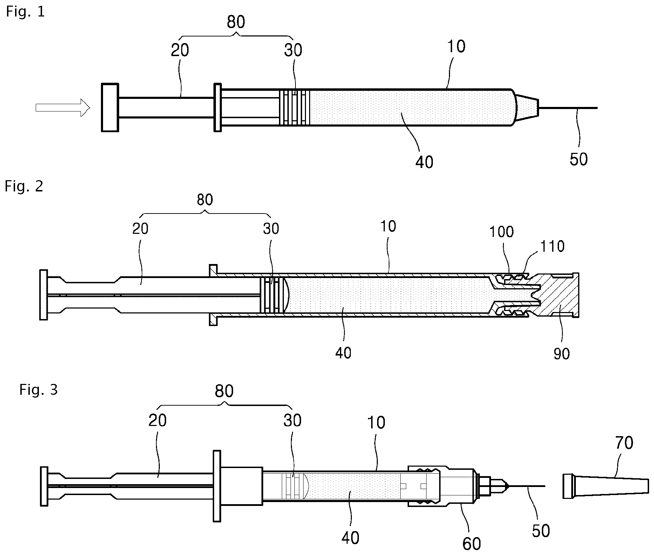

FIG. 1 is a view showing an embodiment of a pre-filled syringe including a needle. The pre-filled syringe may include a barrel 10, a plunger 80 including a plunger rod 20 and a plunger stopper 30, a needle 50, and a botulinum toxin liquid preparation 40 included in a lumen of the barrel 10.

The pre-filled syringe may include a "capping device". The capping device broadly refers to any unit for closing and sealing a distal open outlet end of a syringe. In the present disclosure, the term "open outlet end" or "distal open outlet end" refers to any distal open end of a syringe in fluid-communication with a barrel lumen. The capping device may generally have a channel having a closed end and an open end, which may have dimensions for accommodating and efficiently sealing an open outlet end of a syringe to prevent leakage.

In the case of a pre-filled syringe without a pre-mounted needle, the capping device is a capping unit commonly known as a "tip cap". The tip cap may form a fluid-tight sealing portion with a tip of a syringe, which may efficiently close a syringe barrel and prevent leakage of contents of the syringe barrel. The tip cap is usually removably coupled to the syringe tip or luer collar. The luer collar may surround an upper part of a syringe barrel (for example, a syringe tip). The luer collar may have an internal thread, and the tip cap may have an external thread complementary to the internal thread of the luer collar, in order to couple the tip cap to the syringe barrel. In the case of a pre-filled syringe of the present disclosure, the lure collar may be generally integrally formed with the syringe barrel. Prior to use, the tip cap may be removed, and then a needle cannula (a needle assembly) may be positively coupled to the syringe tip.

FIG. 2 is a view showing an embodiment of a pre-filled syringe including a tip cap. The pre-filled syringe may include the barrel 10, the plunger 80 including the plunger rod 20 and the plunger stopper 30, a tip 100 formed at a terminus of the barrel, and a luer collar 110 formed at the tip and having threads, a tip cap 120 fixed to the tip by coupling with the luer collar, and the botulinum toxin liquid preparation 40 included in the lumen of the barrel 10.

When the pre-filled syringe includes a removable or non-removable (i.e., permanent) cannula or needle cannula (also referred to as a "needle" or "needle assembly") that extends from the syringe tip to deliver the botulinum toxin preparation from the syringe, the capping device may be referred to as a "needle shield". The needle shield may generally have a channel having a closed end and an open end, which may have dimensions for accommodating and coupling a cannula (needle) mounted on the tip of the syringe. Typically, a (sharp) end of the cannula may penetrate the closed end of the channel in the needle shield to seal the open end of the cannula.

FIG. 3 is a view showing an embodiment of a pre-filled syringe including a needle assembly and a needle shield. The pre-filled syringe may include a barrel 10, a plunger 80 including a plunger rod 20 and a plunger stopper 30, a needle 50, a needle assembly 60, a needle shield 70, and a botulinum toxin liquid preparation 40 included in a lumen of the barrel 10.

The capping device (e.g., tip cap or needle shield) may be a single member and may usually be prepared by using a flexible and elastic polymer material (elastomer), or may have a rigid plastic material coupled to a flexible and elastic inner cap, or for example, may be an outer cap prepared by using an elastomer or a material made of elastomer, wherein at least a portion thereof may contact and seal the distal opening of the syringe. In general, at least outlet coupling portion that may form a fluid-tight sealing portion in contact with the distal tip opening may be prepared from a flexible and/or elastic material (e.g., an elastomer), and the binding portion may be in contact with the botulinum toxin preparation during storage and/or use. The outlet coupling portion may be prepared of a material that minimizes possibility of undesired extractables/leachables. In order to further reduce an amount of extractables and/or leachables and to increase compatibility with the botulinum toxin preparation, the outlet coupling portion may have a coating thereon.

Suitable flexible and/or elastic materials of the capping device, in particular the outlet coupling portion, may include an elastomer that may enable long-term storage without interfering with an aqueous botulinum toxin preparation. In particular, a portion of a sealing device, e.g., an outlet coupling portion, configured to contact or in contact with the botulinum toxin formulation is required to exhibit low levels of extractables/leachables during long-term storage of the botulinum toxin preparation. The term "elastomer" or "elastomer material" as used herein may include a crosslinked thermosetting rubbery polymer that is more easily deformable than plastics but is approved for use with a pharmacy-grade fluid and is not easily leached or gas-transferred.

The elastomer material may be isoprene rubber (IS), butadiene rubber (polybutadiene, BR), butyl rubber (copolymer of isobutylene and isoprene, IIR), halogenated butyl rubber (e.g., chlorobutyl rubber, CIIR; and bromobutyl rubber, BIIR), styrene-butadiene rubber (copolymer of styrene and butadiene, SBR), or a mixture thereof. For example, the elastomer material may be butyl rubber or halogenated butyl rubber, in particular bromobutyl rubber or chlorobutyl rubber, or a mixture thereof. The elastomer material may also be reinforced by an inert mineral. In addition, the elastomer material may be cured, for example, by organic peroxides, phenolic resins, and the like.

Suitable coatings that may selectively be present on the outlet coupling portion prepared from the elastomer materials mentioned above generally do unsuitably interfere with the aqueous botulinum toxin preparation and may be prepared by using a material that exhibits a low level of extractables/leachables. The coating used in the present disclosure may include polypropylene, polyethylene, parylene (e.g., parylene N, parylene C, and parylene HT), crosslinked silicone, or fluoropolymer coating. Examples of suitable crosslinked silicone coatings include B2-coating (Daikyo Seiko) or XSi^{™} (Becton Dickinson).

The fluoropolymer coating may be a fluorinated ethylene-propylene copolymer (e.g., a tetrafluoroethylene-hexafluoropropylene copolymer (FEP)), a fluorinated ethylene-ethylene copolymer (e.g., ethylene tetrafluoroethylene copolymer (ETFE), such as Flurotec^{®}), PVA (a copolymer of tetrafluoroethylene (TFE) and perfluoropropyl vinyl ether (PPVE)), a tetrafluoroethylene-perfluoroethylene copolymer, a polyvinylidene fluoride (PVDF), a polyvinyl fluoride (PVF), polytetrafluoroethylene (PTFE), or a mixture thereof. The coating may be prepared, for example, by using ETFE, and may be a Flurotec^{®} coating.

The botulinum toxin liquid preparation may be an aqueous preparation. The aqueous preparation may include an aqueous suspension, an aqueous dispersion, an aqueous emulsion, or an aqueous solution.

A concentration of botulinum toxin in the botulinum toxin liquid preparation may be, for example, about 1 to about 3,000 U/mL, about 10 U/mL to about 1,000 U/mL, about 10 U/mL to about 400 U/mL, about 10 U/mL to about 200 U/mL, about 10 U/mL to about 100 U/mL, about 10 U/mL to about 70 U/mL, about 20 U/mL to about 60 U/mL, or about 40 U/m L.

An amount of the botulinum toxin liquid preparation may be 10 to 80, 10 to 50, 20 to 80, 20 to 60, 20 to 50, 30 to 80, 40 to 80, 20 to 40, or 25 to 35 unit/syringe.

The term "botulinum toxin" as used herein broadly refers to any form or type of botulinum toxin. More specifically, the botulinum toxin may be botulinum toxin types A, B, C1, C2, D, E, F, G, or a mixture thereof. For example, the botulinum toxin may be serotypes A, B, or C1.

In addition, the term "botulinum toxin" is intended to include both a botulinum toxin complex ("toxin complex") and a "neurotoxic component" of the botulinum toxin or complex. The term "botulinum toxin complex" as used herein refers to a high molecular weight complex including a non-toxic protein of *Clostridium botulinum,* including a neurotoxic component of approximately 150 kDa and also a hemagglutinin and a nonhemagglutinin protein. The botulinum toxin serotype A complex may be, for example, Meditoxin (available from Medytox).

The term "neurotoxic component" as used herein relates to a neurotoxic polypeptide ("150 kDa" polypeptide; usually in its 2-chain form) of a toxin complex, without any associated non-toxic proteins. The pure neurotoxic component may be, for example, Coretox (available from Medytox). For example, the term "botulinum toxin" refers to the neurotoxic component of a botulinum toxin complex of a given serotype (e.g., serotypes A, B, or C1, in particular serotype A). In other words, the botulinum toxin formulation contained in the pre-filled syringe may contain only the neurotoxic component and may be free of any other protein of the *Clostridium botulinum* toxin complex.

The botulinum toxin may include functional (i.e., biologically active) isoforms, homologs, orthologs, paralogs, or fragments of botulinum toxin, showing sequence identity of at least 50 %, at least 60 %, at least 70 %, at least 80 %, or at least 90 % to the amino acid sequence of a neurotoxic component of wild-type botulinum toxin, for example, wild-type botulinum toxin A deposited in the GenBank database with Accession No. AAA23262 or botulinum toxin of serotype A1.

Sequence identity may be calculated by any algorithm suitable for producing reliable results, for example, using FASTA algorithm (WR Pearson & DJ Lipman, PNAS 85:2444-2448, 1988). Sequence identity may be calculated by comparing 2 polypeptides or 2 domains, such as 2 LC domains or fragments thereof.

The botulinum toxin may include modified and recombinant botulinum toxins.

The botulinum toxin liquid preparation may include other pharmaceutically acceptable substances such as salts (e.g., sodium chloride), stabilizing proteins (e.g., albumin or gelatin), sugars (e.g., glucose, fructose, galactose, trehalose, sucrose, and maltose), carbohydrate polymers (e.g., hyaluronic acid and polyvinylpyrrolidone (PVP)), polyols (e.g., sugar alcohols such as mannitol, inositol, lactitol, isomalt, xylitol, erythritol, or sorbitol and glycerol), amino acids, vitamins (e.g., vitamin C), zinc, magnesium, anesthetics (e.g., local anesthetics such as lidocaine), surfactants, tonicity modifiers, and the like. The term "pharmaceutically acceptable" as used herein refers to compounds or substances suitable for contact with mammals, particularly human tissues.

The botulinum toxin liquid preparation may not contain any animal component. The botulinum toxin liquid preparation may not include albumin. For example, the botulinum toxin liquid preparation may include botulinum toxin, amino acids, surfactants, and a tonicity adjuster in water. For example, the botulinum toxin liquid preparation may include 10 U/mL to 70 U/mL of botulinum toxin, 0.05 g/L to 0.7 g/L of amino acids, and 0.10 g/L to 0.5 g/L of surfactants, and 0.10 g/L to 1.0 g/L of NaCl in water. The botulinum toxin may be botulinum toxin types A, B, C1, C2, D, E, F, G, or a mixture thereof. The amino acid may be glycine, alanine, valine, leucine, isoleucine, proline, phenylalanine, tyrosine, tryptophan, serine, threonine, cysteine, methionine, asparazine, glutamine, lysine, arginine, histidine, aspartic acid, glutamic acid, a pharmaceutically acceptable salt thereof, or a mixture thereof. The amino acid may be, for example, methionine, histidine, arginine, a pharmaceutically acceptable salt thereof, or a mixture thereof. The surfactant may be a nonionic surfactant. The nonionic surfactant may be polysorbate, poloxamer, or a mixture thereof. The polysorbate may be polysorbate 20, polysorbate 40, polysorbate 60, polysorbate 80, or a mixture thereof. The NaCl may be added at a physiologically acceptable concentration, for example, from about 0.85 g/L to 0.95 g/L, or about 0.9 g/L.

The botulinum toxin liquid preparation may further contain a buffer to maintain a pH of about 5.5 to about 7.5. The buffer may be citrate, histidine, HEPES, arginine, acetic acid, phosphoric acid, a salt thereof, or a mixture thereof.

A pH of the botulinum toxin preparation may be from about 5.5 to about 7.5, about 6.0 to about 7.5, about 6.5 to about 7.5, about 6.1 to about 7.3, or about 6.2 to about 7.2 during storage.

Upon storage for 2, 4, or 6 months at 25 °C, which is an accelerated test condition, the botulinum toxin liquid preparation may show an LD₅₀ titer recovery rate of 75 % to 125 %, 75 % to 120 %, or 80 % to 125 % relative to an initial value.

Upon storage for 2, 4, or 6 months at 25 °C, which is an accelerated test condition, the botulinum toxin liquid preparation may show a protease activity recovery rate of 75 % to 125 %, 75 % to 120 %, or 80 % to 125 % relative to an initial value.

Upon storage for 2, 4, or 6 months at 25 °C, which is an accelerated test condition, the botulinum toxin liquid preparation may show a pH change of ± 1.5, ±1.0, or ± 0.5 or less relative to an initial value.

In an embodiment, a syringe formulation pre-filled with botulinum toxin may be provided, wherein the syringe formulation may include a syringe including a syringe barrel, a plunger rod, and a plunger stopper, and a botulinum toxin liquid preparation filled in the syringe, wherein an amount of increase of a force acting on an end of the plunger rod may decrease as a rate of discharge of the botulinum toxin liquid preparation increases.

In an embodiment, a ratio of a length to an inner diameter of the syringe barrel may be 10 to 22. The inner diameter of the barrel of the syringe may be in a range of 3.5 mm to 6.5 mm, and the length of the barrel may be in a range of 60 mm to 100 mm. The material of the syringe barrel may be glass, COC, or silicone-coated COP. The material of the syringe barrel may be COC. The silicone coating may be spray-on silicone coating, baked-on silicone coating, or polydimethylsiloxane coating. A material of the plunger stopper may be isoprene rubber (IS), butadiene rubber (BR), butyl rubber, halogenated butyl rubber, styrene-butadiene rubber, or a mixture thereof. The plunger stopper may be uncoated. A dose of the botulinum toxin liquid preparation may be 10 unit/syringe to 50 unit/syringe. The botulinum toxin liquid preparation may not include albumin. The botulinum toxin liquid preparation may not contain any animal component. The botulinum toxin liquid preparation may include botulinum toxin, amino acids, surfactants, and an isotonic agent. The amino acid may be methionine. The surfactant may be polysorbate 20, polysorbate 40, polysorbate 60, polysorbate 80, or poloxamer. The botulinum toxin liquid preparation may further contain a buffer to maintain a pH of 5.5 to 7.5. The buffer may be citrate, histidine, HEPES, arginine, acetic acid, phosphoric acid, a salt thereof, or a mixture thereof. Upon storage for 2, 4, or 6 months at 25 °C, which is an accelerated test condition, the botulinum toxin liquid preparation may show an LD₅₀ titer recovery rate of 80 % to 125 % relative to an initial value. Upon storage for 2, 4, or 6 months at 25 °C, which is an accelerated test condition, the botulinum toxin liquid preparation may show a protease activity recovery rate of 80 % to 125 % relative to an initial value. Upon storage for 2, 4, or 6 months at 25 °C, which is an accelerated test condition, the botulinum toxin liquid preparation may show a pH change of ± 1.0 or less relative to an initial value.

In an embodiment, a syringe formulation pre-filled with botulinum toxin may be provided, wherein the syringe formulation may include a syringe including a syringe barrel, a plunger rod, and a plunger stopper, and a botulinum toxin liquid preparation filled in the syringe, wherein a material of the syringe barrel may be glass, COC, or silicone-coated COP.

In an embodiment, the material of the syringe barrel may be COC. The silicone coating may be spray-on silicone coating, baked-on silicone coating, or polydimethylsiloxane coating. A material of the plunger stopper may be isoprene rubber (IS), butadiene rubber (BR), butyl rubber, halogenated butyl rubber, styrene-butadiene rubber, or a mixture thereof. The plunger stopper may be uncoated. An amount of increase of a force acting on an end of the plunger rod may decrease as a rate of discharge of the botulinum toxin liquid preparation increases. A ratio of a length to an inner diameter of the syringe barrel may be 10 to 22. The inner diameter of the barrel of the syringe may be in a range of 3.5 mm to 6.5 mm, and the length of the barrel may be in a range of 60 mm to 100 mm. A dose of the botulinum toxin liquid preparation may be 10 unit/syringe to 50 unit/syringe. The botulinum toxin liquid preparation may not include albumin. The botulinum toxin liquid preparation may not contain any animal component. The botulinum toxin liquid preparation may include botulinum toxin, amino acids, surfactants, and an isotonic agent. The amino acid may be methionine. The surfactant may be polysorbate 20, polysorbate 40, polysorbate 60, polysorbate 80, or poloxamer. The botulinum toxin liquid preparation may further contain a buffer to maintain a pH of 5.5 to 7.5. The buffer may be citrate, histidine, HEPES, arginine, acetic acid, phosphoric acid, a salt thereof, or a mixture thereof. Upon storage for 2, 4, or 6 months at 25 °C, which is an accelerated test condition, the botulinum toxin liquid preparation may show an LD₅₀ titer recovery rate of 80 % to 125 % relative to an initial value. Upon storage for 2, 4, or 6 months at 25 °C, which is an accelerated test condition, the botulinum toxin liquid preparation may show a protease activity recovery rate of 80 % to 125 % relative to an initial value. Upon storage for 2, 4, or 6 months at 25 °C, which is an accelerated test condition, the botulinum toxin liquid preparation may show a pH change of ± 1.0 or less relative to an initial value.

In an embodiment, a syringe formulation pre-filled with botulinum toxin may be provided, wherein the syringe formulation may include a syringe including a syringe barrel, a plunger rod, and a plunger stopper, and a botulinum toxin liquid preparation filled in the syringe, wherein the botulinum toxin liquid preparation may not include albumin.

In this embodiment, a syringe formulation pre-filled with botulinum toxin may be provided, wherein the syringe formulation may include a syringe including a syringe barrel, a plunger rod, and a plunger stopper, and a botulinum toxin liquid preparation filled in the syringe, wherein the botulinum toxin liquid preparation may not include any animal component. The botulinum toxin liquid preparation may include botulinum toxin, amino acids, surfactants, and an isotonic agent. The amino acid may be methionine. The surfactant may be polysorbate 20, polysorbate 40, polysorbate 60, polysorbate 80, or poloxamer. The botulinum toxin liquid preparation may further contain a buffer to maintain a pH of 5.5 to 7.5. The buffer may be citrate, histidine, HEPES, arginine, acetic acid, phosphoric acid, a salt thereof, or a mixture thereof. A dose of the botulinum toxin liquid preparation may be 10 unit/syringe to 50 unit/syringe. The material of the syringe barrel may be glass, COC, or silicone-coated COP. The material of the syringe barrel may be COC. The silicone coating may be spray-on silicone coating, baked-on silicone coating, or polydimethylsiloxane coating. A material of the plunger stopper may be isoprene rubber (IS), butadiene rubber (BR), butyl rubber, halogenated butyl rubber, styrene-butadiene rubber, or a mixture thereof. The plunger stopper may be uncoated. In the syringe formulation pre-filled with botulinum toxin, an amount of increase of a force acting on an end of the plunger rod may decrease as a rate of discharge of the botulinum toxin liquid preparation increases. A ratio of a length to an inner diameter of the syringe barrel may be 10 to 22. The inner diameter of the barrel of the syringe may be in a range of 3.5 mm to 6.5 mm, and the length of the barrel may be in a range of 60 mm to 100 mm. Upon storage for 2, 4, or 6 months at 25 °C, which is an accelerated test condition, the botulinum toxin liquid preparation may show an LD₅₀ titer recovery rate of 80 % to 125 % relative to an initial value. Upon storage for 2, 4, or 6 months at 25 °C, which is an accelerated test condition, the botulinum toxin liquid preparation may show a protease activity recovery rate of 80 % to 125 % relative to an initial value. Upon storage for 2, 4, or 6 months at 25 °C, which is an accelerated test condition, the botulinum toxin liquid preparation may show a pH change of ± 1.0 or less relative to an initial value.

### ADVANTAGEOUS EFFECTS OF DISCLOSURE

Upon storage for 2, 4, or 6 months at 25 °C, which is an accelerated test condition, an LD₅₀ titer, a botulinum toxin endopeptidase titer, and pH of a botulinum toxin pre-filled syringe formulation according to an aspect may be stably maintained. In addition, a discharge rate of the botulinum toxin pre-filled syringe formulation may be easily controlled.

### BRIEF DESCRIPTION OF DRAWINGS

FIG. 1 is a view showing an embodiment of a pre-filled syringe including a needle.
FIG. 2 is a view showing an embodiment of a pre-filled syringe including a tip cap.
FIG. 3 is a view showing an embodiment of a pre-filled syringe including a needle assembly and a needle shield.

### MODE OF DISCLOSURE

Hereinafter, the present disclosure will be described in more detail with reference to Examples. However, these Examples are for illustrative purposes only, and the present disclosure is not intended to be limited by these Examples.

### Example 1: Stability of botulinum toxin pre-filled liquid syringe formulation: syringe having a barrel made of COC or COP material

### 1. Preparation of botulinum toxin pre-filled liquid preparation

In this Example, a syringe (hereinafter referred to as an 'HVD syringe') was filled with a liquid botulinum toxin preparation for use in a highly viscous drug (HVD), and stability according to the storage period was measured. The HVD syringe had an inner diameter of 5.00 mm, an outer diameter of 9.40 mm, a barrel length of 80.0 mm, and a volume filled with a liquid preparation of 0.8 mL. For reference, as an example of a known syringe, TopPac^{™} syringe available from SCHOTT has an inner diameter of 6.50 mm, an outer diameter of 9.40 mm, a barrel length of 64.5 mm, and a volume filled with a liquid preparation of 1 mL. That is, the HVD syringe had, as compared with TopPac^{™} syringe, a small inner diameter and a long length. TopPac^{™} syringe included a barrel, a plunger, a plunger rod, and a tip cap, and the barrel was made of a COC material, and a luer-lock was formed. The inner surface of the barrel was siliconized with a reactive silicone mixture and then crosslinked by curing. The tip cap was made of rubber (Datwyler Corporation). The HVD syringe included a COC material made of TOPAS 6015 material and had improved heat resistance.

In addition, in the HVD syringe, stability according to the material of the barrel, the material of the plunger stopper and the material used for the coating thereof, and the composition of the aqueous liquid botulinum toxin preparation filled in the barrel were measured. Here, the syringe included a plastic syringe barrel, a capping device, and a plunger load assembly. The barrel included a proximal end and a distal end, and a wall that may extend therebetween and was generally cylindrical to form a barrel lumen. The barrel had a tip protruding to the distal having a fluid passage extending therethrough and in communication with the barrel lumen, and the wall which was generally cylindrical optionally had an interior surface coated with a barrier layer. In this embodiment, the inner surface of the wall was coated with silicone, that is, the barrier layer was a silicone layer. In addition, in this embodiment, the barrel has a COC or COP material.

The capping device had an outlet coupling portion that sealingly couples and closes the open outlet of the distal end of the barrel. The capping device for the distal end of the barrel consists of a cap and a rubber tip cap, the cap was made of polycarbonate, and the rubber tip cap was made of the same material as the plunger. Here, the rubber tip cap corresponds to the outlet coupling portion. The outlet coupling portion was made of an elastomer material optionally having a coating on the surface. The outlet coupling portion and the capping device are coupled in the form of a lure-lock.

The plunger rod assembly included a plunger stopper extending into the proximal end of the barrel and having sliding fluid-tight engagement with the cylindrical wall of the barrel lumen, wherein the stopper was made of an elastomer material and optionally had a coating on at least a portion of the stopper in contact with the aqueous liquid botulinum toxin preparation during storage and/or injection. In this embodiment, the material of the stopper was bromo butyl rubber (BIIR) or chlorobutyl rubber (CIIR), which is optionally coated with Teflon.

Table 1 shows a botulinum toxin pre-filled syringe preparation of a liquid botulinum toxin preparation in water pre-filled used in this Example.

**[Table 1]**

| Formulation name | A | B | C | D | E | F | G | H |
|---|---|---|---|---|---|---|---|---|
| Barrel material | COC | COC | COC | COC | COC | COC | COP | COP |
| Plunger stopper material | BIIR | BIIR | BIIR | BIIR | CIIR | CIIR | CIIR | CIIR |
| Plunger stopper coating | Not progr essed | Not progre ssed | Teflon | Teflon | Not progress ed | Not progress ed | Teflon | Teflon |
| L-Met (g/L) | 0.1 | 0.2 | 0.1 | 0.2 | 0.1 | 0.2 | 0.1 | 0.2 |
| T20 (g/L) | 0.3 | 0.15 | 0.30 | 0.15 | 0.3 | 0.15 | 0.3 | 0.15 |
| NaCl (g/L) | 9 | 9 | 9 | 9 | 9 | 9 | 9 | 9 |
| Toxin concentration (U/mL) | 40 | 40 | 40 | 40 | 40 | 40 | 40 | 40 |
| Water-filling volume (mL) | 0.8 | 0.8 | 0.8 | 0.8 | 0.8 | 0.8 | 0.8 | 0.8 |

In Table 1, COC represents cyclic olefin copolymer, and COP represents a cyclic olefin polymer. BIIR and CIIR represent bromobutyl rubber and chlorobutyl rubber (IIR), respectively. The plunger stopper material represents the material of the rubber part. Here, the butyl rubber (IRR) is a copolymer of isobutylene and isoprene. Teflon coating indicates a coating of FluroTec^{R} film on the BIIR or CIIR surface. L-Met, T20, NaCl, and toxin respectively represent the amount of L-methionine, polysorbate 20, NaCl, and botulinum toxin type A component dissolved in water, i.e., injectable water.

The syringe preparation was to couple the plunger rod assembly into the lumen of the barrel such that the stopper of the plunger sealed the liquid preparation while in contact with the liquid preparation. In addition, the outlet coupling portion of the distal end of the barrel was sealed with the capping device to close the open outlet. The syringe and the capping device were integral, and the capping device was removed immediately before use. At this time, the liquid comes into contact with the rubber tip cap inside the capping device.

That is, the pre-filled syringe botulinum liquid preparation was in a state in which the syringe was not coupled to the needle and bound to the capping device.

As a result, the material of the syringe barrel was selected from COC and COP, and the stopper of the plunger was selected from bromo butyl rubber and chlorobutyl rubber and a Teflon coating thereof in 4 syringes in which concentrations of methionine and polysorbate 20 were varied twice, to prepare a total of 8 pre-filled syringe botulinum liquid preparations.

### 2. Long-term stability test of pre-filled syringe formulation

### (1) Titer test

The eight prepared formulations, i.e., Formulations A to H, were stored at 4 °C in a stable sample storage room to measure half lethal doses (LD₅₀) of the mice at 0, 3, 6, 9, 12, 18, and 24 months.

The liquid samples of the 8 formulations were each serial diluted and administered to the abdominal cavity of 4-week-old female ICR mice weighing 17 g to 22 g. The fatality rate for each diluted sample was observed for 3 days after administration, and the lethal doses 50 were obtained using the statistical program PROBIT. The half lethal doses at 0 months was set to 100 %, and the tendency to change in half lethal doses obtained at each point in time was expressed in %. Table 2 shows half lethal doses according to the storage period of Formulations A, B, C, D, E, F, G, and H.

**[Table 2]**

| LD₅₀ (recovery lethal dose with respect to initial value, %) | | | | | | | |
|---|---|---|---|---|---|---|---|
| Name | Tested periods (months) | | | | | | |
| | 0 | 3 | 6 | 9 | 12 | 18 | 24 |
| A | 100 | 99 | 94 | 89 | 83 | 77 | 75 |
| B | 100 | 96 | 90 | 90 | 92 | 89 | 85 |
| C | 100 | 96 | 75 | 95 | 86 | 76 | 73 |
| D | 100 | 102 | 75 | 84 | 86 | 77 | 77 |
| E | 100 | 100 | 73 | 91 | 97 | 79 | 77 |
| F | 100 | 96 | 76 | 80 | 80 | 78 | 78 |
| G | 100 | 97 | 99 | - | - | - | - |
| H | 100 | 79 | 92 | - | - | - | - |

As shown in Table 2, the recovery lethal dose of Formulations A to F at 24 months was measured at least 73 %, but the recovery lethal dose of Formulations G and H were not measured from 9 months. This indicates that when the barrel is made of a COC material and coated with silicone, regardless of whether the plunger stopper material is BIIR or CIIR or whether the surface of the plunger stopper is Teflon-coated, the botulinum toxin is stable for 24 months. When the barrel material is COP, the recovery titer was not measured in the syringe after 9 months.

### (2) Botulinum toxin endopeptidase titer analysis

The eight pre-filled syringe Formulations A to H were stored in a stability chamber to measure activity of BoNTA/LC at 0, 3, 6, 9, 12, 18, and 24 months.

100 µL of SNAP25 (self-produced) fragments consisting of 70 amino acid sequences were added to each well of the 96-well plate and incubated at 2 °C to 8 °C for 16 hours. The SNAP25 fragment includes 10 or more consecutive amino acid sequences, including the cleavage site between amino acids No. 197 and No. 198.

BoNTA samples, which are standard samples from which quantitative curves may be obtained using BoNTA standards (Innotox available from Medytox), were prepared, and the test samples were prepared by diluting BoNTA in HEPES solution (available from Sigma) so that the result values of the Formulations A to H samples could be included in the quantitative curve range. The standard sample and each of the test samples A to H were placed in a well containing SNAP25 and reacted at room temperature for 1 hour. Next, an anti-SNAP25 polyclonal antibody (self-produced) was added to each well to react at room temperature for 1 hour. The HRP-bound secondary antibody was reacted at room temperature for 1 hour, and then the color reagent 3,3',5,5'-tetramethylbenzidine (TMB) was added and incubated to induce a color reaction.

The absorbance was measured at 450 nm in a plate reader (Microplate Reader available from TECAN) to quantify the degree of color development according to the activity of BoNT/LC, and the quantitative curve was obtained using the absorbance value of the standard sample. The absorbance values of Samples A to H were substituted into the quantitative curve to calculate the activity of BoNT/LC of each sample. The activity of BoNT/LC at 0 months was set to 100 %, and the tendency to change in activity of BoNT/LC obtained at each point in time was expressed in %. Table 3 shows the endopeptidase activity of BoNTA included in Formulations A to H according to the storage period.

**[Table 3]**

| Protease activity (recovered activity relative to initial value, %) | | | | | | | |
|---|---|---|---|---|---|---|---|
| Name | Tested periods (months) | | | | | | |
| | 0 | 3 | 6 | 9 | 12 | 18 | 24 |
| A | 100 | 106 | 109 | 103 | 121 | 123 | 141 |
| B | 100 | 102 | 105 | 103 | 119 | 115 | 130 |
| C | 100 | 107 | 111 | 101 | 127 | 107 | 124 |
| D | 100 | 100 | 104 | 94 | 121 | 101 | 118 |
| E | 100 | 99 | 107 | 92 | 119 | 99 | 111 |
| F | 100 | 108 | 104 | 96 | 130 | 111 | 130 |
| G | 100 | 107 | 112 | - | - | - | - |
| H | 100 | 100 | 109 | - | - | - | - |

As shown in Table 3, the recovery titers of Formulations A to F at 24 months was maintained 111 % to 141 %, but the recovery titers of Formulations G and H were not measured from 9 months.

### (3) pH stability

At 0, 3, 6, 9, 12, 18, and 24 months of the eight pre-filled syringe Formulations A to H, each sample was placed in a 15 mL disposable tube to be at least 3 mL, and the pH of each sample was measured by using a pH meter (available from Mettler Toledo). Table 4 shows the pH changes of Formulations A to H with the storage period.

**[Table 4]**

| pH | | | | | | | |
|---|---|---|---|---|---|---|---|
| Name | Tested periods (months) | | | | | | |
| | 0 | 3 | 6 | 9 | 12 | 18 | 24 |
| A | 6.7 | 6.9 | 7.0 | 7.3 | 7.2 | 7.3 | 7.4 |
| B | 6.8 | 7.1 | 7.1 | 7.3 | 7.3 | 7.3 | 7.3 |
| C | 6.0 | 6.5 | 6.6 | 6.6 | 6.5 | 6.5 | 6.8 |
| D | 6.1 | 6.5 | 6.5 | 6.5 | 6.5 | 6.6 | 6.8 |
| E | 6.3 | 6.6 | 6.5 | 6.6 | 6.5 | 6.7 | 6.8 |
| F | 6.2 | 6.6 | 6.5 | 6.6 | 6.7 | 6.7 | 6.6 |
| G | 5.9 | 5.7 | 5.5 | - | - | - | - |
| H | 5.9 | 5.6 | 5.3 | - | - | - | - |

As shown in Table 4, the pH of Formulations A to F were maintained between 6.8 and 7.4 at 24 months, but the pH of Formulations G and H were maintained between 5.3 to 5.9 at 6 months when activity was measured.

### Example 2: Stability of botulinum toxin pre-filled liquid syringe formulation: syringe having a barrel made of glass or COP material

### 1. Preparation of botulinum toxin pre-filled liquid preparation

Botulinum toxin pre-filled liquid preparations as shown in Table 5 were prepared. Here, the syringe was the HVD syringe shown in Table 1, which had an inner diameter of 5.00 mm, an outer diameter of 9.40 mm, a barrel length of 80.0 mm, and a volume filled with a liquid preparation of 0.8 mL.

**[Table 5]**

| Formulatio n name | I | J | K | L | M | N |
|---|---|---|---|---|---|---|
| Barrel material | Glass | Glass | COP | COP | COP | COP |
| Plunger stopper material | BIIR | BIIR | BIIR | BIIR | i-coating | i-coating |
| L-Met (g/L) | 0.05 | 0.1 | 0.05 | 0.1 | 0.05 | 0.1 |
| T20 (g/L) | 0.3 | 0.3 | 0.3 | 0.3 | 0.3 | 0.3 |
| NaCl (g/L) | 9 | 9 | 9 | 9 | 9 | 9 |
| Toxin concentration (U/mL) | 40 | 40 | 40 | 40 | 40 | 40 |
| Water-filling volume (mL/syringe) | 0.8 | 0.8 | 0.8 | 0.8 | 0.8 | 0.8 |

In Table 5, the barrel of Formulations I and J was available from POONGLIM Pharmatech Inc. (catalog number, Art.69, Korea), and the barrel of Formulations K and L was SiO₂ Medical Products Inc. (Catalog No., 850009-100-04, USA), and the barrel of Formulations M and N was a product of Terumo Corporation (Catalog No., PJ-B1L9BFTF1, Japan). The botulinum toxin was BTX1301 (API batch number) from Medytox, BoNTA. In Table 5, COC represents cyclic olefin copolymer, and COP represents a cyclic olefin polymer. BIIR and CIIR represent bromobutyl rubber and chlorobutyl rubber (IIR), respectively. Here, the butyl rubber (IRR) is a copolymer of isobutylene and isoprene. L-Met, T20, NaCl, and toxin respectively represent the amount of L-methionine, polysorbate 20, NaCl, and botulinum toxin type A component dissolved in water. The liquid containing these components had a pH of 6.0 to 7.0.

The preparation was to couple the plunger rod assembly into the lumen of the barrel such that the stopper of the plunger sealed the liquid preparation while in contact with the liquid preparation. In addition, the outlet coupling portion of the distal end of the barrel was sealed with the capping device to close the open outlet. That is, the pre-filled syringe botulinum liquid preparation was in a state in which the syringe was not coupled to the needle and bound to the capping device.

As a result, the material of the syringe barrel was selected from glass and COP, and the stopper of the plunger was selected from bromo butyl rubber and i-coating in 3 syringes in which concentrations of methionine and polysorbate 20 were varied twice, to prepare a total of 6 pre-filled syringe botulinum Liquid Preparations I to N.

### 2. Stability test of pre-filled Syringe Formulation I to N

### (1) Titer test

The prepared 6 formulations, i.e., Formulations I to N samples, were stored at 25 °C in a thermohygrostat (available from Binder Inc.) for experimentation under accelerated test conditions. For long-term storage tests, the sample was stored at 4 °C in a stable sample storage room. The half lethal dose of mice at 0, 2, 3, 4, and 6 months after storage (LD₅₀) was measured.

The liquid samples of the 6 formulations were each serial diluted and administered to the abdominal cavity of 4-week-old female ICR mice weighing 17 g to 22 g. The fatality rate for each diluted sample was observed for 3 days after administration, and the lethal doses 50 were obtained using the statistical program PROBIT. The half lethal doses at 0 months was set to 100 %, and the tendency to change in half lethal doses obtained at each point in time was expressed in %. Table 6 shows half lethal doses according to the storage period of Formulations I, J, K, L, M, and N.

**[Table 6]**

| LD₅₀ (recovery lethal dose with respect to initial value, %) | | | | | | |
|---|---|---|---|---|---|---|
| Name | | Tested periods (months) | | | | |
| | | 0 | 2 | 3 | 4 | 6 |
| I | Accelerate d | 100 | 97 | - | 87 | 89 |
| | Long-term | 100 | - | 93 | - | 94 |
| J | Accelerate d | 100 | 98 | - | 86 | 89 |
| | Long-term | 100 | - | 89 | - | 94 |
| K | Accelerate d | 100 | 100 | - | 90 | 97 |
| | Long-term | 100 | - | 92 | - | 97 |
| L | Accelerate d | 100 | 105 | - | 97 | 102 |
| | Long-term | 100 | - | 100 | - | 108 |
| M | Accelerate d | 100 | 100 | - | 92 | 91 |
| | Long-term | 100 | - | 96 | - | 102 |
| N | Accelerate d | 100 | 93 | - | 85 | 86 |
| | Long-term | 100 | - | 90 | - | 93 |

As shown in Table 6, at 6 months, the titer of each of the six pre-filled syringe Formulations I to N maintained in the range of 86 % to 108 %, as compared with the initial titer.

### (2) Botulinum toxin endopeptidase titer analysis

The six pre-filled syringe Formulations I to N were stored in a stability chamber to measure activity of BoNTA/LC at 0, 2, 3, 4, and 6 months.

100 µL of SNAP25 (self-produced) fragments consisting of 70 amino acid sequences were added to each well of the 96-well plate and incubated at 2 °C to 8 °C for 16 hours. The SNAP25 fragment includes 10 or more consecutive amino acid sequences, including the cleavage site between amino acids No. 197 and No. 198.

BoNTA samples, which are standard samples from which quantitative curves may be obtained using BoNTA standards (Innotox available from Medytox), were prepared, and the test samples were prepared by diluting BoNTA in HEPES solution (available from Sigma) such that the resulting values of the Formulations A to H samples were included in the quantitative curve range. The standard sample and each of the test samples A to H were placed in a well containing SNAP25 and reacted at room temperature for 1 hour. Next, an anti-SNAP25 polyclonal antibody (self-produced) was added to each well to react at room temperature for 1 hour. The HRP-bound secondary antibody was reacted at room temperature for 1 hour, and then the color reagent 3,3',5,5'-tetramethylbenzidine (TMB) was added and incubated to induce a color reaction.

The absorbance was measured at 450 nm in a plate reader (Microplate Reader available from TECAN) to quantify the degree of color development according to the activity of BoNT/LC, and the quantitative curve was obtained using the absorbance value of the standard sample. The absorbance values of Samples I to N were substituted into the quantitative curve to calculate the activity of BoNT/LC of each sample. The activity of BoNT/LC at 0 months was set to 100 %, and the tendency to change in activity of BoNT/LC obtained at each point in time was expressed in %. Table 7 shows the endopeptidase activity of BoNTA included in Formulations I to N according to the storage period.

**[Table 7]**

| Protease activity (recovered activity relative to initial value, %) | | | | | | |
|---|---|---|---|---|---|---|
| Name | | Tested periods (months) | | | | |
| | | 0 | 2 | 3 | 4 | 6 |
| I | Accelerated | 100 | 81 | - | 54 | 53 |
| | Long-term | 100 | - | 79 | - | 80 |
| J | Accelerated | 100 | 68 | - | 67 | 65 |
| | Long-term | 100 | - | 73 | - | 83 |
| K | Accelerated | 100 | 76 | - | 63 | 70 |
| | Long-term | 100 | - | 84 | - | 83 |
| L | Accelerated | 100 | 78 | - | 82 | 78 |
| | Long-term | 100 | - | 85 | - | 90 |
| M | Accelerated | 100 | 82 | - | 79 | 81 |
| | Long-term | 100 | - | 92 | - | 96 |
| N | Accelerated | 100 | 86 | - | 83 | 84 |
| | Long-term | 100 | - | 92 | - | 94 |

As shown in Table 7, the recovery titers of Formulations I to N at 6 months remained at 53 % to 94 %, as compared to the initial value.

### (3) pH stability

At certain points in time of the six pre-filled syringe Formulations I to N, each sample was placed in a 15 mL disposable tube to be at least 3 mL, and the pH of each sample was measured by using a pH meter (available from Mettler Toledo). Table 8 shows the pH changes of Formulations I to N with the storage period. The point in time was 0, 2, 4, and 6 months for the accelerated condition experiment, and 0, 3, and 6 months for the long-term preservation experiment.

**[Table 8]**

| pH | | | | | | |
|---|---|---|---|---|---|---|
| Name | | Tested periods (months) | | | | |
| | | 0 | 2 | 3 | 4 | 6 |
| I | Accelerated | 6.5 | 7.8 | | 7.3 | 7.9 |
| | Long-term | 6.5 | | 7.6 | | 7.7 |
| J | Accelerated | 6.7 | 7.8 | | 7.4 | 7.8 |
| | Long-term | 6.7 | | 7.6 | | 7.8 |
| K | Accelerated | 6.6 | 7.6 | | 7.2 | 7.6 |
| | Long-term | 6.6 | | 7.4 | | 7.5 |
| L | Accelerated | 6.5 | 7.5 | | 7.1 | 7.4 |
| | Long-term | 6.5 | | 7.2 | | 7.4 |
| M | Accelerated | 6.2 | 7.4 | | 6.7 | 7.3 |
| | Long-term | 6.2 | | 7.2 | | 7.3 |
| N | Accelerated | 5.9 | 7.3 | | 6.7 | 7.1 |
| | Long-term | 5.9 | | 7.1 | | 7.1 |

As shown in Table 8, the pH of Formulations I to N was maintained at 7.1 to 7.9 until 6 months.

### Example 3: Stability of botulinum toxin pre-filled liquid syringe formulation: Effects of albumin

### 1. Preparation of botulinum toxin pre-filled liquid preparation

As shown in Table 9, Formulation O containing human serum albumin and not containing L-Met and polysorbate 20 and Formulation P containing L-Met and polysorbate 20 and not containing human serum albumin were prepared. In Table 9, the solvent was water, and Toxin was a BoNTA type available from Medytox Corporation.

The HVD syringes were filled with Formulations O and P. Here, the HVD syringe is Syringe B in Table 1, the barrel was made of a COC material, and the plunger material was made of siliconized BIIR.

**[Table 9]**

| Name | NaCl | L-Met | Tween20 | HSA | Toxin |
|---|---|---|---|---|---|
| | 0.9 % | 0.02 % | 0.015 % | 0.02 % | 30 Unit) |
| O | + | - | - | + | + |
| P | + | + | + | - | + |

### 2. Titer test: LD50 (accelerated test)

Syringe formulations with which a syringe was pre-filled with the two prepared types, i.e., samples of Formulations O and P, were stored in a stability chamber, and the half lethal dose (LD50) of mice at 0, 2, 3, 7, 14, 28, and 56 days was measured.

Samples 1 and 2 were each serial diluted in water and administered to the abdominal cavity of 4-week-old female ICR mice weighing 17 g to 22 g. The fatality rate for each diluted sample was observed for 3 days after administration, and the lethal doses 50 were obtained using the statistical program PROBIT. The half lethal doses at 0 months was set to 100 %, and the tendency to change in half lethal doses obtained at each point in time was expressed in %. The shelf life was calculated using the Arenius equation (https://met.uk.com/medical-device-packaging-testing/4a-medical-accelerated-ageing), which shows the relationship between the reaction rate constant and temperature.

Table 10 shows the storage stability of Formulation O containing albumin and the storage stability of Formulation P not containing albumin are shown in LD₅₀ values.

**[Table 10]**

| Stability test period (days) | | 0 | 2 | 5 | 7 | 14 | 28 | 56 |
|---|---|---|---|---|---|---|---|---|
| LD50 (%) | STD | 97 | 97 | 103 | 101 | 103 | 105 | 96 |
| | O | 92 | 68 | 50 | 48 | 25 | N/A | N/A |
| | P | 109 | 98 | 91 | 94 | 86 | 86 | 52 |
| Normalizatio n | O | 100 | 74 | 55 | 53 | 28 | N/A | N/A |
| | P | 100 | 90 | 84 | 87 | 79 | 79 | 48 |
| Valid period (days) | | 0 | 30 | 60 | 90 | 170 | 340 | 680 |

In Table 10, STD represents Meditoxin (available from Medytox) as the standard sample, normalization represents relative titers when the data value is set to 100 at day 0, and N/A represents that the experiment was not performed because the test result LD50 value in the previous cycle was very low at 20 %, and it is not meaningful to proceed with the test. At 56 days of testing, that is, about 2 months, of three formulations prepared, i.e., the standard sample (STD), Formulation O, and Formulation P, under accelerated conditions, the recovery titer was lost at 28 days of testing in the case of Formulation O containing albumin, whereas the recovery titer of Formulation P not containing albumin was maintained at 86 % at 28 days and at 52 % at 56 days. The accelerated conditions were by storing the samples at 40 °C and recovering the samples at the time of 0, 2, 5, 7, 14, 28, and 56 days.

### 3. Injection force test

Formulation O containing albumin and Formulation P not containing albumin as shown in Table 9 were respectively filled in 1 mL of SHOTT TopPac syringe and HVD syringe, i.e., B syringe in Table 1, as described in Section 1 of Example 1, thereby preparing four types of formulations, i.e., pre-filled syringe botulinum Liquid Preparations Q, R, S, and T.

For the four types of formulations, a MultiTest 2.5 (Mecmesin, UK) tensile compression apparatus was used to measure a plunger injection force according to the plunger movement rate of the syringe. The injection force is an average injection force.

Specifically, 30 G needles were first coupled to a syringe containing each of the 4 formulations. Next, the syringe was fixed to the jig of the tensile compression apparatus and adjusted such that the plunger rod was centered on the load cell of the apparatus. The jig is a syringe fixing part of the apparatus.

After setting the measuring distance in consideration of the length of each syringe, the test was performed by entering the speed value in the program embedded in the apparatus and pressing the start button to activate the apparatus. In case of the HVD syringe, due to a small inner diameter and a long length, when filling with 1 mL, the contents were filled to a height of about 40 mm, and in the case of the TopPac syringe, when filling with 1 mL, the contents were filled to a height of about 30 mm. Thus, when measuring the injection force, the measuring distance was set to have a margin of 5 mm. Thus, the measuring distance for the HVD syringe was from 0 mm to 35 mm, and the measuring distance for the TopPac syringe was from 0 mm to 25 mm, to press the plunger. The sample that was measured was removed from the jig and measured 3 times repeatedly per sample to obtain the injection force result. Table 11 shows the injection force according to the plunger movement rate.

**[Table 11]**

| Name | | Q | R | S | T |
|---|---|---|---|---|---|
| Preparation | | O | O | P | P |
| Syringe type | | TopPac 1 mL | HVD 1 mL | TopPac 1 mL | HVD 1 mL |
| Average plunger injection force (N) | 10 mm/min | 1.45 | 1.68 | 1.13 | 1.35 |
| | 50 mm/min | 2.72 | 2.72 | 2.52 | 2.53 |
| | 100 mm/min | 4.24 | 3.79 | 3.08 | 3.30 |
| | 200 mm/min | 6.25 | 4.72 | 4.84 | 4.39 |

As shown in Table 11, Formulations R and T including the HVD syringes had the same or higher injection force, as compared with Formulations Q and S including the TopPac syringes at a low plunger movement rate of 10 and 50 mm/min, but the injection forces of Formulations R and T were lower than those of Formulations Q and S including the TopPac syringes at a high plunger movement rate of 100 and 200 mm/min. This indicates that, as compared with Formulations Q and S including the TopPac syringes, Formulations R and T including the HVD syringes have a high plunger compression force at a low plunger movement rate and have a low plunger compression force at a high plunger movement rate. Therefore, formulations including the HVD syringes may be finely manipulated at low speeds and easier to inject at high speeds.

## Claims

1. A syringe formulation pre-filled with botulinum toxin, comprising: a syringe comprising a syringe barrel, a plunger rod, and a plunger stopper; and a botulinum toxin liquid preparation filled in the syringe, wherein an amount of increase of a force acting on an end of the plunger rod decreases as a rate of discharge of the botulinum toxin liquid preparation increases.

2. The syringe formulation of claim 1, wherein a ratio of a length to an inner diameter of the syringe barrel is 10 to 22.

3. The syringe formulation of claim 2, wherein the inner diameter of the syringe barrel is 3.5 mm to 6.5 mm, and the length of the syringe barrel is 60 mm to 100 mm.

4. The syringe formulation of claim 1, wherein a material of the syringe barrel is glass, COC, or COP.

5. The syringe formulation of claim 1, wherein a material of the plunger stopper is isoprene rubber (IS), butadiene rubber (BR), butyl rubber, halogenated butyl rubber, styrene-butadiene rubber, or a mixture thereof.

6. The syringe formulation of claim 1, wherein the botulinum toxin liquid preparation does not contain albumin.

7. The syringe formulation of claim 1, wherein the botulinum toxin liquid preparation does not contain any animal component.

8. The syringe formulation of claim 6 or 7, wherein the botulinum toxin liquid preparation comprises botulinum toxin, an amino acid, a surfactant, and an isotonic agent.

9. The syringe formulation of claim 8, wherein the amino acid is methionine.

10. The syringe formulation of claim 8, wherein the surfactant is polysorbate 20, polysorbate 40, polysorbate 60, polysorbate 80, or poloxamer.

11. The syringe formulation of claim 6 or 8, wherein the botulinum toxin liquid preparation further contains a buffer to maintain a pH of 5.5 to 7.5.

12. The syringe formulation of claim 11, wherein the buffer is citrate, histidine, HEPES, arginine, acetic acid, phosphoric acid, a salt thereof, or a mixture thereof.

13. The syringe formulation any one of claims 1 to 8, wherein, upon storage for 2, 4, or 6 months at 25 °C, which is an accelerated test condition, the botulinum toxin liquid preparation shows an LD₅₀ titer recovery rate of 80 % to 125 % relative to an initial value.

14. The syringe formulation any one of claims 1 to 8, wherein, upon storage for 2, 4, or 6 months at 25 °C, which is an accelerated test condition, the botulinum toxin liquid preparation shows a protease activity recovery rate of 80 % to 125 % relative to an initial value.

15. The syringe formulation any one of claims 1 to 8, wherein, upon storage for 2, 4, or 6 months at 25 °C, which is an accelerated test condition, the botulinum toxin liquid preparation shows a pH change of ± 1.0 or less relative to an initial value.
